# FASCICULE DE BREVET EUROPEEN

(11) **EP 0 950 049 B1**
(45) Date de publication et mention de la délivrance du brevet: **17.10.2001**
(21) Numéro de dépôt: 97951326.4
(22) Date de dépôt: 12.12.1997
(51) Int. Cl.: C07D 211/70, C07D 401/04, A61K 31/445

(54) **DIPHENYLALKYL-TETRAHYDROPYRIDINES, PROCEDE POUR LEUR PREPARATION ET COMPOSITION PHARMACEUTIQUE LES CONTENANT**
DIPHENYLALKYL-TETRAHYDROPYRIDINE, VERFAHREN ZU IHREN HERSTELLUNG UND DIESE ENTHALTENDE ARZNEIMITTEL
DIPHENYL ALKYL-TETRAHYDROPYRIDINES, PROCESS FOR THEIR PREPARATION, AND PHARMACEUTICAL COMPOSITIONS CONTAINING THEM

(30) Priorité: 13.12.1996 FR 9615336
(43) Date de publication de la demande: 20.10.1999
(73) Titulaire: SANOFI-SYNTHELABO, 75013 Paris (FR)
(72) Inventeur: BARONI, Marco, I-20010 Vanzago (IT); CARDAMONE, Rosanna, I-22100 Como (IT); FOURNIER, Jacqueline, F-31830 Plaisance du Touch (FR); GUZZI, Umberto, I-20148 Milan (IT)
(74) Mandataire: Gillard, Marie-Louise
(86) Numéro de dépôt international: FR9702289
(87) Numéro de publication internationale: WO9825904

(56) Documents cités:
- EP-A- 0 412 901
- EP-A- 0 458 696
- WO-A-93/11107
- DE-A- 2 158 077

## Description

La présente invention concerne de nouvelles gem-diphénylalkyl-1,2,3,6-tétrahydropyridines 4-substituées ayant une activité neurotrophique et neuroprotectrice, un procédé pour leur préparation, et des compositions pharmaceutiques les contenant.

EP-0 458 696 décrit l'utilisation d'une 1-(2-naphtyléthyl)-4-(3-trifluorométhylphényl)-1,2,3,6-tétrahydropyridine pour la préparation de médicaments destinés au traitement de troubles cérébraux et neuronaux.

WO 93/11107 décrit des dérivés de 1,2,3,6-tétrahydropyridine N-hydroxy-substituée utiles pour le traitement de l'hypoxie et de l'ischérnie.

EP-0 412 901 décrit l'utilisation de trifluorométhylphényltétrahydropyridines pour la préparation de médicaments destinés à combattre les troubles de la motricité intestinale.

DE-21 58 077 décrit des dérivés de 1-(3,3-diphénylpropyl)pipéridine ayant une activité analgésique, antitussive et/ou spasmolytique.

Il a été maintenant trouvé que certaines gem-diphénylalkyl-1,2,3,6-tétrahydropyridines, substituées par un groupe phényle ou pyridyle, exercent une action neurotrophique sur le système nerveux semblable à celle du facteur de croissance nerveuse (NGF de l'anglais Nerve Growth Factor) et peuvent rétablir le fonctionnement des cellules endommagées ou présentant des anomalies dans leurs fonctions physiologiques.

La présente invention concerne donc, selon un de ses aspects, les gem-diphénylalkyl-1,2,3,6-tétrahydropyridines de formule (I) 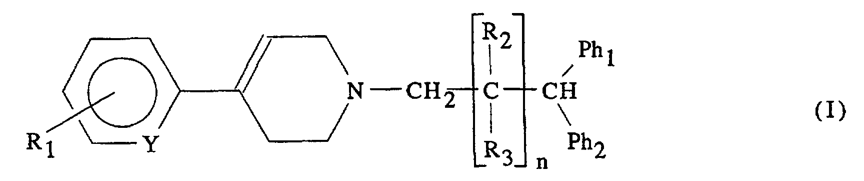 dans laquelle
- Y: représente -CH- ou -N-;
- R₁: représente un halogène, un groupe CF₃, (C₁-C₄)alkyle ou (C₁-C₄)alcoxy;
- R₂ et R₃: représentent chacun l'hydrogène ou un (C₁-C₃)alkyle;
- n: représente 0 ou 1
- Ph₁ et Ph₂: représentent chacun, indépendemment, un groupe phényle non substitué, mono- ou polysubstitué,
ainsi que leurs sels et solvates et leurs sels d'ammonium quaternaire.
Des composés avantageux selon la présente invention, sont ceux de formule (I) dans laquelle Y est CH et R₁ est CF₃ et ceux de formule (I) dans laquelle Y est N et R₁ est Cl.
Parmi ces composés, ceux de formule (I) où, en plus, R₂ et R₃ sont tous les deux des hydrogènes et n est 0 ou 1, sont particulièrement avantageux.
Des composés particulièrement avantageux sont représentés par la formule (I') 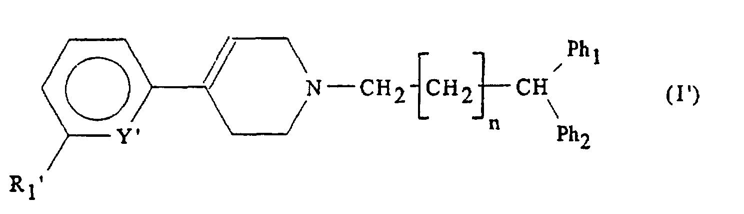 dans laquelle R₁' est CF₃ et Y' est CH ou bien R₁' est Cl et Y' est N, et n est zéro ou 1, Ph₁ et Ph₂ étant tels que définis ci-dessus.

Dans les formules (I) et (I'), Ph₁ et Ph₂ sont de préférence identiques, mais ils peuvent également être différents, ce qui implique l'existence d'un atome de carbone chiral.

Avantageusement, Ph₁ et Ph₂ représentent chacun indépendemment un groupe phényle ; un groupe phényle monosubstitué en position 2, 3 ou 4 par un atome de fluor, de chlore ou par un groupe méthyle, éthyle, *n*-propyle, *i*-propyle, *n*-butyle, *i*-butyle, *s*-butyle, *t*-butyle, trifluorométhyle, cyano, méthoxy, méthylthio, méthylsulfonyle, éthoxy, éthylthio, éthylsulfonyle, (C₁-C₃)alcoxycarbonyle ou di(C₁-C₃)alkylaminocarbonyle ; un groupe phényle disubstitué dans les positions 2, 4; 3, 4; 3, 5 ou 2, 6 par un atome de fluor, de chlore, ou par un groupe méthyle, éthyle, trifluorométhyle, cyano ou méthoxy ; ou un groupe phényle trisubstitué dans les positions 3, 4, 5; 2, 4, 5 ou 2, 4, 6 par un atome de chlore, de fluor, ou par un groupe méthyle, éthyle, trifluorométhyle, cyano ou méthoxy.

Lorsque Ph₁ et Ph₂ sont différents, un des groupes phényle est de préférence non substitué et l'autre est de préférence monosubstitué dans la position 2, 3 ou 4 comme indiqué ci-dessus.

Dans la présente description, le terme "(C₁-C₃)alkyle" désigne les groupes méthyle, éthyle, *n*-propyle et *i*-propyle.

Le terme "alcoxy" désigne un groupe hydroxyle substitué par un groupe (C₁-C₄)alkyle.

Des composés particulièrement préférés sont les suivants:
- la 1-(2,2-diphényléthyl)-4-(3-trifluorométhylphényl)-1,2,3,6-tétrahydropyridine;
- la 1-[2,2-(4,4'-dichlorodiphényl)éthyl]-4-(3-trifluorométhylphényl)-1,2,3,6-tétrahydropyridine;
- la 1-[2,2-(3,3'-bistrifluorométhyldiphényl)éthyl]-4-(3-trifluorométhylphényl)-1,2,3,6-tétrahydropyridine;
- la 1-[2,2-(4,4'-diméthoxydiphényl)éthyl]-4-(3-trifluorométhylphényl)-1,2,3,6-tétrahydropyridine;
- la 1-[2-(4-fluorophényl)-2-phényléthyl]-4-(3-trifluorométhylphényl)-1,2,3,6-tétrahydropyridine;
- la 1 -(3,3-diphénylpropyl)-4-(3-trifluorométhylphényl)-1,2,3,6-tétrahydropyridine;
- la 1-[2,2-(4,4'-dichlorodiphényl)éthyl]-4-(6-chloropyrid-2-yl)-1,2,3,6-tétrahydropyridine;
et leurs sels, solvates ou sels d'ammonium quaternaire.

Selon un autre de ses aspects, la présente invention concerne un procédé pour la préparation des composés de formule (I), de leurs sels ou solvates et de leurs sels d'ammonium quaternaire, caractérisé en ce que
(a) on fait réagir une aryl-1,2,3,6-tétrahydropyridine de formule (II) 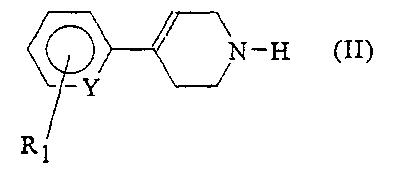 dans laquelle Y et R₁ sont tels que définis ci-dessus, avec un acide de formule (III) 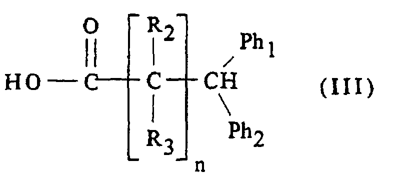 dans laquelle n, R₂, R₃, Ph₁ et Ph₂ sont tels que définis ci-dessus, ou l'un de ses dérivés foncionnels,
(b) on réduit le carbonyle intermédiaire de formule (IV), 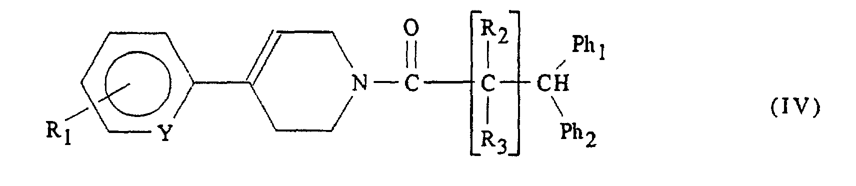 et
(c) on isole le composé de formule (I) ainsi obtenu et, éventuellement on le transforme en l'un de ses sels ou solvates ou l'un de ses sels d'ammonium quaternaire.

La réaction de l'étape (a) peut être convenablement conduite dans un solvant organique à une température comprise entre -10°C et la température de reflux du mélange réactionnel ; de préférence la réaction est conduite à basse température.

Comme solvant de réaction, on utilise de préférence un solvant halogéné tel que le chlorure de méthylène, le dichloroéthane, le 1,1,1-trichloroéthane, le chloroforme et similaires ou un alcool, tel que le méthanol ou l'éthanol, mais d'autres solvants organiques compatibles avec les réactifs employés, par exemple le dioxane, le tétrahydrofurane ou un hydrocarbure tel que l'hexane, peuvent être également employés.

La réaction peut être convenablement conduite en présence d'un accepteur de protons, par exemple d'un carbonate alcalin ou d'une amine tertiaire. Comme dérivé fonctionnel approprié de l'acide de formule (III), on peut utiliser l'acide libre, éventuellement activé (par exemple avec le BOP), l'anhydride, un anhydride mixte, un ester activé ou un halogénure d'acide, de préférence le chlorure ou bromure. Parmi les esters activés, l'ester de *p*-nitrophényle est particulièrement préféré, mais les esters de méthoxyphényle, de trityle, de benzhydryle et similaires sont également convenables.

La réduction de l'étape (b) peut être convenablement réalisée par des agents de réduction appropriés tels que les hydrures d'aluminium ou un hydrure complexe de lithium et d'aluminium dans un solvant organique inerte à une température comprise entre 0°C et la température de reflux du mélange réactionnel, selon les techniques usuelles.

Par "solvant organique inerte" on entend un solvant qui n'interfère pas avec la réaction. De tels solvants sont par exemple les éthers, tel que l'éther diéthylique, le tétrahydrofurane, le dioxane ou le 1,2-diméthoxyéthane.

Le composé de formule (I) obtenu est isolé selon les techniques usuelles et éventuellement transformé en l'un de ses sels d'addition d'acides ou, lorsqu'un groupe acide est présent, le caractère amphotère du composé permet la séparation des sels soit avec des acides soit avec des bases.

Lorsque les sels du composé de formule (I) sont préparés pour être administrés en tant que médicaments, il faut que les acides ou les bases employées soient pharmaceutiquement acceptables; si l'on prépare des sels du composé de formule (I) dans un autre but, par exemple pour mieux purifier le produit ou pour mieux effectuer des essais analytiques, on peut alors utiliser n'importe quel acide ou base.

Les sels avec des acides pharmaceutiquement acceptables sont par exemple, ceux avec les acides minéraux, tels que le chlorhydrate, le bromhydrate, le borate, le phosphate, le sulfate, l'hydrogénosulfate, l'hydrogénophosphate, le dihydrogénophosphate et ceux avec les acides organiques, tels que le citrate, le benzoate, l'ascorbate, le méthylsulfate, le naphtalène-2-sulfonate, le picrate, le fumarate, le maléate, le malonate, l'oxalate, le succinate, l'acétate, le tartrate, le mésylate, le tosylate, l'iséthionate, l'α-cétoglutarate, l'α-glycérophosphate, le glucose-1-phosphate, etc.

Les sels avec des bases pharmaceutiquement acceptables sont par exemple ceux avec les métaux alcalins ou alcalino-terreux, tels que le sodium, le potassium, le calcium, le magnésium et ceux avec les bases organiques, telles que les amines, les acides aminés basiques (la lysine, l'arginine, l'histidine), le trométamol, la N-méthylglutamine, etc.

Les amines de départ de formule (II) où Y est CH sont des composés connus ou bien elles peuvent être préparées selon des procédés analogues à ceux utilisés pour préparer les composés connus.

Les amines de départ de formule (II) où Y est N peuvent être préparées par réaction de la 2-halogénopyridine appropriée de formule (p) 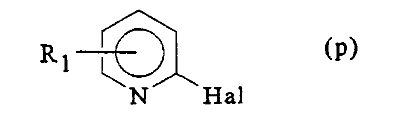 dans laquelle R₁ est tel que défini ci-dessus et Hal est un atome d'halogène,
avec une 1,2,3,6-tétrahydropyridine de formule (q) 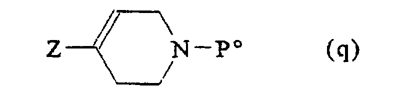 dans laquelle P° représente un groupe protecteur tel que par exemple le groupe benzyle et Z représente un substituant qui permet la substitution nucléophile de l'halogène de la pyridine. De tels substituants sont par exemple les trialkylstannanes, comme le tributylstannane ou les composés de Grignard.

On déprotège ensuite la 1,2,3,6-tétrahydropyridine par clivage du groupe protecteur dans des conditions convenables.

Les acides de formule (III) où n est 1 peuvent être préparés, selon la réaction de Wittig, par
a) réaction entre une benzophénone appropriée de formule (r) 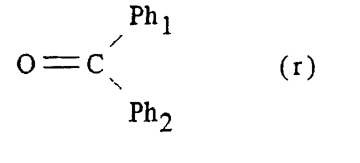 dans laquelle Ph₁ et Ph₂ sont tels que définis ci-dessus et un composé de formule (s) 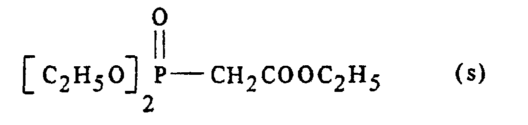 selon la réaction de Wittig (comme décrit par exemple dans J. Med. Chem, 1996, 39(1 1):2197-2206), suivie par
b) la réduction catalytique de l'intermédiaire de formule (t) 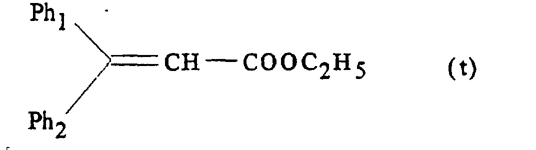 en présence d'un catalyseur tel que le Pd/C, et
c) l'alkylation éventuelle de l'intermédiaire de formule (u) 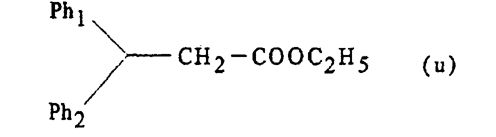 dans la position α par rapport au carbonyle de l'ester selon les méthodes connues lorsqu'on souhaite préparer des composés de formule (I) où R₂ et R₃ sont autres que l'hydrogène et l'hydrolyse de l'ester de formule (v) 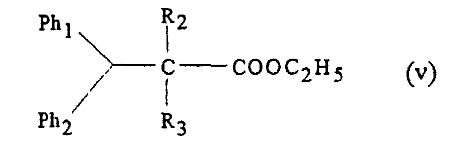 pour obtenir l'acide de formule (III) où n est 1.

Les acides de formule (III) où n est 0 peuvent être préparés à partir de la benzophénone appropriée de formule (r) 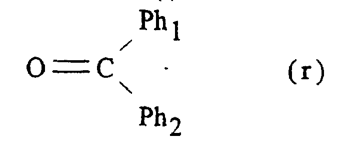 dans lequel Ph₁ et Ph₂ sont tels que définis ci-dessus, par réaction avec l'iodure de triméthylsulfoxonium et oxydation de l'aldéhyde intermédiaire de formule (w) 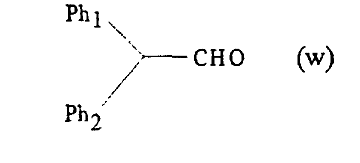 selon la méthode décrite dans J. Am. Chem. Soc., 1990, 112(18):6690-6695 pour obtenir l'acide correspondant.
Selon un autre mode opératoire, les composés de formule (I) où n est 0 peuvent également être préparés par réaction entre une aryl-1,2,3,6-tétrahydropyridine de formule (II) 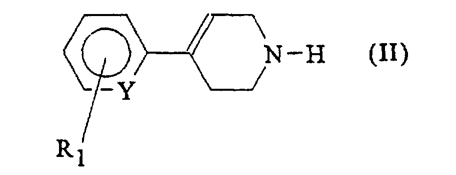 dans laquelle R₁ et Y sont tels que définis ci-dessus,
et un aldéhyde de formule (w) ci-dessus en présence d'un agent de réduction tel que le cyanoborohydrure de sodium, selon les techniques connues.

L'activité des composés de formule (I) sur le système nerveux a été demontrée dans des études i*n vitro* et *in vivo* selon les méthodes décrites dans EP-0 458 696 et, pour l'évaluation de la survie neuronale, à l'aide d'un test de survie *in vitro* conduit en utilisant des neurones isolés à partir de dissections de la région septale d'embryons de rats.

Selon cé test, on préleve la région septale d'embryons de rats agés de 17-18 jours sous microscope à dissection dans des conditions stériles, puis on la dissocie dans un milieu trypsine-EDTA. La suspension de cellules est placée dans un flacon de culture dans un milieu DME/Ham's F12 (v:v) (Dulbecco Modified Eagle Medium/ Nutrient Mixture Ham's F12 - R.G. Ham, Proc. Nat. Sci, 1965, 53:288) contenant du sérum de veau à 5% et du sérum de cheval à 5% et maintenue à 37°C pendant 90 minutes. Ce traitement permet l'élimination des cellules non-neuronales.

Les neuroblastes sont ensuite ensemencés dans les puits d'une plaque de titration à raison de 17x10⁴ cellules/cm², dans un milieu de culture non sérique constitué par du DME/Ham's F12 contenant du sélénium (30 nM) et de la transferrine (1,25 µM). Chaque puits a été préalablement traité à la poly-L-lysine. Les plaques ensemencées sont placées dans un incubateur à l'étuve (37°C; 5% CO₂).

Les composés à tester sont dissous dans du DMSO et dilués comme requis par le milieu de culture.

Les neuroblastes sont maintenus dans des plaques contenant le composé à tester ou le solvant correspondant pendant 4 jours sans changer le milieu.

Après 4 jours le milieu est remplacé par un sel de tétrazolium dissous dans le milieu de culture (0,15 mg/ml). Les cellules sont ensuite placées à l'étuve à 37°C pendant 4 heures. Les succinodéshydrogénases mitochondriales des cellules vivantes réduisent le sel de tétrazolium en bleu formazan dont, après dissolution dans le DMSO, on mesure la densité optique à 540 nm. Cette densité est linéairement corrélée au nombre de cellules vivantes (Manthorpe et al., Dev. Brain Res., 1988, 25:191-198).

La différence entre les groupes contenant les composés à tester et les témoins a été évaluée par analyse statistique en utilisant le test t bilatéral de Dunnett ("two-tailed Dunnett t-test").

Dans ce dernier test les composés de formule (I) se sont montrés aussi actifs ou plus actifs que les composés décrits dans EP-0 458 696, l'efficacité de certains composés de formule (I) vis-à-vis de la survie neuronale étant double par rapport au composé A décrit dans EP-0 458 696.

Grâce à cette puissante activité neuroprotectrice, et à leur faible toxicité compatible pour une utilisation en tant que médicaments, les composés de formule (I) ainsi que leurs sels d'addition pharmaceutiquement acceptables, leurs solvates et leurs sels d'ammonium quaternaire sont utilisables pour la préparation de compositions pharmaceutiques indiquées dans le traitement et/ou la prophylaxie de toutes les maladies qui impliquent une dégénérescence neuronale. Plus particulièrement, les composés de l'invention sont utilisables, seuls ou en co-administration ou association avec d'autres principes actifs agissant sur le SNC, par exemple les inhibiteurs de l'acétylcholinestérase, les cholinomimétiques M1 sélectifs, les antagonistes NMDA, les nootropiques tels que le piracétam, notamment dans les indications suivantes: troubles de la mémoire, démence vasculaire, troubles post-encéphalitiques, troubles post-apoplectiques, syndromes post-traumatiques dus à un traumatisme crânien, troubles dérivant d'anoxies cérébrales, maladie d'Alzheimer, démence sénile, démence subcorticale, telle que la chorée de Huntington et la maladie de Parkinson, démence provoquée par le SIDA, neuropathies dérivées de morbidité ou dommage des nerfs sympathiques ou sensoriels, et maladies cérébrales, telles que l'oedème cérébral, et les dégénérescences spinocérébelleuses, les dégénérescences des motoneurones, comme par exemple la sclérose latérale amyotrophique.

L'administration des composés de l'invention peut être convenablement effectuée par voie orale, parentérale, sublinguale ou transdermique. La quantité de principe actif à administrer dans le traitement des troubles cérébraux et neuronaux selon la méthode de la présente invention dépend de la nature et de la gravité des affections à traiter ainsi que du poids des malades. Néanmoins, les doses unitaires préférées comprendront généralement de 0,25 à 700 mg, avantageusement de 0,5 à 300 mg, de préférence de 1 à 150 mg, par exemple entre 2 et 50 mg, à savoir 2, 5, 10, 15, 20, 25, 30, 40 ou 50 mg, de produit. Ces doses unitaires seront administrées normalement une ou plusieurs fois par jour, par exemple 2, 3, 4, ou 5 fois par jour, de préférence une à trois fois par jour, la dose globale chez l'homme étant variable entre 0,5 et 1400 mg par jour, avantageusement entre 1 et 900 mg par jour, par exemple de 2 à 500 mg, plus convenablement de 2 à 200 mg par jour.

Selon un autre de ses aspects, la présente invention a pour objet une composition pharmaceutique contenant, en tant que principes actifs, un composé de formule (I) ci-dessus et un composé indiqué dans le traitement symptomatique de la démence sénile du type Alzheimer (DAT) ou leurs sels pharmaceutiquement acceptables.

L'expression "composé indiqué dans le traitement symptomatique de la démence sénile du type Alzheimer (DAT)" indique un produit qui est capable d'améliorer le cadre symptomatologique des patients atteints par la DAT sans intervenir sur les causes de la maladie.

De tels composés sont par exemple les inhibiteurs de l'acétylcholinestérase, les agonistes muscariniques M₁, les agonistes nicotiniques, les antagonistes du récepteur NMDA, les nootropiques.

Des inhibiteurs de l'acétylcholinestérase préférés sont le donépézil et la tacrine.

D'autres inhibiteurs de l'acétylcholinestérase pouvant être utilisés sont par exemple la rivastigmine (SDZ-ENA-713), la galanthamine, le métrifonate, l'eptastigmine, la veinacrine, la physostigmine (Drugs, 1997, 53(5): 752-768; The Merck Index 12 ed.).

D'autres inhibiteurs de l'acétylcholinestérase sont encore la 5,7-dihydro-3-[2-[1-(phénylméthyl)-4-pipéridinyl]éthyl]-6H-pyrrolo[3,2-f]-1,2-benzisoxazol-6-one nommée aussi icopezil (J. Med. Chem., 1995, 38: 2802-2808), le MDL-73,745 ou zifrosilone (Eur. J. Pharmacol., 1995, 276: 93-99), le TAK-147 (J. Med. Chem., 1994, 37: 2292-2299).

D'autres inhibiteurs de l'acétylcholinestérase sont par exemple ceux qui sont décrits dans les demandes de brevet JP 09-095483, WO 97/13754, WO 97/21681, WO 97/19929, ZA 96-04565, US 5,455,245, WO 95-21822, EP 637 586, US 5,401,749, EP 742 207, US 5,547,960, WO 96/20176, WO 96/02524, EP 677 516, JP 07-188177, JP 07-133274, EP 649 846, EP 648 771, JP 07-048370, US 5,391,553, WO 94/29272, EP 627 400.

Selon un autre de ses aspects, la présente invention concerne une composition pharmaceutique contenant, en tant que principes actifs, un composé de formule (I) et un agoniste du récepteur M₁, ou leurs sels pharmaceutiquement acceptables.

Des agonistes du récepteur M₁ sont par exemple la milaméline, la bésipiridine, la talsaclidine, la xanoméline, le YM-796 et le YM-954 (Eur. J. Pharmacol., 1990, 187: 479-486), la 3-[N-(2-diéthylamino-2-méthylpropyl)-6-phényl-5-propyl]-pyridazinamine, nommée aussi SR-46559 (Biorg. Med. Chem. Let., 1992, 2: 833:838), le AF-102, le CI-979, le L-689, 660, le LU 25-109, le S-99 77-2, le SB 202,026, la thiopilocarpine, le WAL 2014 (Pharmacol. Toxicol., 1996, 78: 59-68).

Selon un autre de ses aspects, l'invention concerne une composition pharmaceutique contenant, en tant que principes actifs, un composé de formule (I) et un agoniste nicotinique ou leurs sels pharmaceutiquement acceptables.

Des agonistes nicotiniques avantageux sont par exemple le MKC-231 (Biorg. Med. Chem. Let., 1995, 5 (14): 1495-1500), le T-588 (Japan J. Pharmacol., 1993, 62: 81-86), le ABT-418 (Br. J. Pharmacol., 1997, 120: 429-438).

Selon un autre de ses aspects, l'invention concerne une composition pharmaceutique contenant, en tant que principes actifs, un composé de formule (I) et un antagoniste des récepteurs NMDA ou leurs sels pharmaceutiquement acceptables.

Un antagoniste des récepteurs NMDA avantageux est par exemple la mémantine (Arzneim. Forsch., 1991, 41: 773-780).

Selon un autre de ses aspects, l'invention concerne une composition pharmaceutique contenant, en tant que principes actifs un composé de formule (I) et un agent nootropique, ou leurs sels pharmaceutiquement acceptables.

Des agents nootropiques qui peuvent être utilisés selon l'invention sont par exemple le nétiracétam, le nébracétarn (Merck Index, 12^{th} ed.).

Les doses des deux principes actifs associés sont choisies en général parmi les doses qui seraient administrées pour chaque médicament dans le traitement non combiné.

Selon un aspect ultérieur, la présente invention concerne aussi une méthode de traitement de la démence sénile du type Alzheimer qui consiste à administrer à un patient atteint de cette maladie une dose efficace d'un composé de formule (I) ou d'un de ses sels pharmaceutiquement acceptables et une dose efficace d'un composé indiqué dans le traitement symptomatique de la DAT ou d'un de ses sels pharmaceutiquement acceptables, lesdites administrations étant simultanées, séquentielles ou étalées dans le temps et les doses efficaces des principes actifs pouvant être contenues dans des formes d'administration unitaires séparées ou bien, lorsque les principes actifs sont administrés simultanément, les deux principes actifs étant avantageusement contenus dans une forme pharmaceutique unique.

Dans les compositions pharmaceutiques de la présente invention pour l'administration orale, sublinguale, sous-cutanée, intramusculaire, intraveineuse, transdermique ou rectale, le principe actif peut être administré sous formes unitaires d'administration, soit tel quel par exemple sous forme lyophilisée, soit en mélange avec des supports pharmaceutiques classiques, aux animaux et aux êtres humains pour le traitement des affections susdites. Les formes unitaires d'administration appropriées comprennent les formes par voie orale telles que les comprimés éventuellement sécables, les gélules, les poudres, les granules et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

Les exemples qui suivent illustrent mieux l'invention sans toutefois la limiter.

### EXEMPLE 1

### 1-(2,2-diphényléthyl)-4-(3-trifluorométhylphényl)-1,2,3,6-tétrahydropyridine et son chlorhydrate

### 1a/ 1-(α,α-diphénylacétyl)-4-(3-trifluorométhylphényl)-1,2,3,6-tétrahydropyridine

A un mélange de 8 g (0,035 mole) de 4-(3-trifluorométhylphényl)-1,2,3,6-tétrahydropyridine, 50 ml de chlorure de méthylène et 4,96 ml de triéthylamine on ajoute goutte à goutte à la température de 0/+5°C, 8 g de chlorure de α,α-diphénylacétyle dans 50 ml de chlorure de méthylène. On agite pendant une heure à la température ambiante, on évapore le solvant sous pression réduite, on réprend le résidu dans de l'éther éthylique, on lave avec une solution aqueuse d'acide chlorhydrique 0,2 M, à l'eau, avec une solution aqueuse de carbonate de sodium et encore à l'eau. On sèche sur du sulfate de sodium, on évapore le solvant sous pression réduite. On obtient 5 g du composé du titre.

### 1b/ 1-(2,2-diphényléthyl)-4-(3-trifluorométhylphényl)-1,2,3,6-tétrahydropyridine et son chlorhydrate

A un mélange de 0,7 g d'hydrure d'aluminium et de lithium dans 10 ml d'éther éthylique, on ajoute goutte à goutte à 25 °C une solution de 5 g (0,012 mole) du produit de l'étape précédente dans 50 ml d'éther éthylique. On agite à la température ambiante pendant une heure, on ajoute goutte à goutte 5 ml d'eau. On sépare les deux phases, on sèche la phase organique sur du sulfate de sodium et on évapore le solvant sous pression réduite. On obtient ainsi la 1-(2,2-diphényléthyl)4-(3-trifluorométhylphényl)-1,2,3,6-tétrahydropyridine. On prépare le chlorhydrate à l'aide d'une solution d'éther éthylique saturée en acide chlorhydrique. On cristallise dans 150 ml d'acétate d'éthyle. P.f. (chlorhydrate) 207-210°C.

### EXEMPLE 2

### 1-[2,2-(4,4'-dichlorodiphényi)éthyl]-4-(3-trifluorométhylphényl)-1,2,3,6-tétrahydropyridine et son oxalate

### 2a/ α,α-(4,4'-dichlorodiphényl)acétaldéhyde

A un mélange de 5,5 g (0,025 mole ) d'iodure de triméthylsulfoxonium dans 10 ml de tétrahydrofuranne anhydre on ajoute par portions 0,75 g (0,025 mole) d'hydrure de sodium à 80% dans l'huile. On chauffe à 55°C pendant 6 heures et on y ajoute 6 g (0,025 mole) de 4,4'-dichlorobenzophénone dans 10 ml de tétrahydrofuranne anhydre. On laisse agiter le mélange à 55 °C pendant une nuit, on verse dans l'eau, on extrait à l'éther éthylique, on sèche la phase organique sur du sulfate de sodium, on évapore le solvant sous pression réduite. On dissout le résidu dans 32 ml de toluène et on y ajoute 3 ml de BF₃-EtO. On agite pendant 2 minutes puis on laisse reposer pendant 3 minutes. On lave deux fois avec une solution aqueuse de bicarbonate de sodium, on sèche la phase organique sur du sulfate de sodium, on évapore le solvant sous pression réduite. On obtient une huile qu'on purifie par chromatographie sur colonne de gel de silice en éluant par un mélange hexanelacétate d'éthyle = 9/1. On obtient le composé du titre.

### 2b/ 1-[2,2-(4,4'-dichlorodiphényl)éthyl]-4-(3-trifluorométhylphényl)-1,2,3,6-tétrahydropyridine et son oxalate

A la température de 0/+5°C on mélange 1,3 g (0,0045 mole) du produit de l'étape précédente, 1,2 g (0,0053 mole) de 4-(3-trifluorométhylphényl)-1,2,3,6-tétrahydropyridine, 21 ml de méthanol, 0,8 ml d'acide acétique glacial et 0,5 g d'acétate de sodium anhydre. A la même température on ajoute au mélange 0,76 g (0,0121 mole) de cyanoborohydrure de sodium, on agite pendant 1,5 heures à basse température, puis à la température ambiante pendant une nuit. On ajoute goutte à goutte 5 ml d'acide chlorhydrique concentré, on laisse agiter pendant 10 minutes, on évapore le méthanol et on reprend le résidu dans un mélange acétate d'éthyle/solution aqueuse de NH₄OH diluée. On sépare les deux phases, on sèche la phase organique sur du sulfate de sodium et on évapore le solvant sous pression réduite. On obtient une huile qu'on purifie par chromatographie sur colonne de gel de silice en éluant par un mélange hexane/acétate d'éthyle = 9/1. On obtient le composé du titre sous forme de base. On prépare l'oxalate à l'aide d'acide oxalique dans de l'isopropanol. P.f. (oxalate) 187-189°C.

### EXEMPLE 3

### 1-[2,2-(3,3'-bistrifluorométhyldiphényl)éthyl]-4-(3-trifluorométhylphényl)-1,2,3,6-tétrahydropyridine et son oxalate

### 3a/α,α-(3,3'-bistrifluorométhyldiphényl)acétaldéhyde

En opérant comme décrit dans l'exemple 2a/, mais en utilisant la 3,3'-bistrifluorométhylbenzophénone on obtient le composé du titre.

### 3b/ 1-[2,2-(3,3'-bistrifluorométhyldiphényl)éthyl]-4-(3-trifluorométhylphényl)-1,2,3,6-tétrahydropyridine et son oxalate

En opérant comme décrit dans l'exemple 2b/, mais en utilisant le produit de l'étape précédente au lieu de l' α,α-(4,4'-dichlorodiphényl)acétaldéhyde on obtient les composés du titre. P.f. (oxalate) 194-196°C.

### EXEMPLE 4

### 1-[2,2-(4,4'-diméthoxydiphényl)éthyl]-4-(3-trifluorométhylphényl)-1,2,3,6-tétrahydropyridine et son chlorhydrate

### 4a/ α,α-(4,4'-diméthoxydiphényl)acétaldéhyde

En opérant comme décrit dans l'exemple 2a/, mais en utilisant la 4,4'-diméthoxybenzophénone on obtient le composé du titre.

### 4b/ 1-[2,2-(4,4'-diméthoxydiphényl)éthyl]-4-(3-trifluorométhylphényl)-1,2,3,6-tétrahydropyridine et son chlorhydrate

En opérant comme décrit dans l'exemple 2b/, mais en utilisant le produit de l'étape précédente au lieu de l' α,α-(4,4'-dichlorodiphényl)acétaldéhyde on obtient les composés du titre. P.f. (chlorhydrate) 214-216°C.

### EXEMPLE 5

### 1-[2-(4-fluorophényl)-2-phényléthyl]-4-(3-trifluorométhylphényl)-1,2,3,6-tétrahydropyridine et son chlorhydrate

### 5a/α-4-fluorophényl-α-phénylacétaldéhyde

En opérant comme décrit dans l'exemple 2a/, mais en utilisant la 4-fluorobenzophénone on obtient le composé du titre.

### 5b/ 1-[2,2-(4-fluorophényl)-2-phényléthyl]-4-(3-trifluorométhylphényl)-1,2,3,6-tétrahydropyridine et son chlorhydrate

En opérant comme décrit dans l'exemple 2b/, mais en utilisant le produit de l'étape précédente au lieu de l' α,α-(4,4'-dichlorodiphényl)acétaldéhyde on obtient les composés du titre. P.f. (chlorhydrate) 206-208°C.

### EXEMPLE 6

### 1-(3,3-diphénylpropyl)-4-(3-trifluorométhylphényl)-1,2,3,6-tétrahydropyridine et son chlorhydrate

En opérant comme décrit dans l'exemple 1b/ mais en utilisant l'acide 3,3-diphénylpropionique commercial (Aldrich, référence D21,165-6) au lieu de l'acide 2,2-diphénylacétique, on obtient les composés du titre. P.f. (chlorhydrate) 176-178°C.

### EXEMPLE 7

### 1-[2,2-(4,4'-dichlorodiphényl)éthyl]-4-(6-chloropyrid-2-yl)-1,2,3,6-tétrahydropyridine et son chlorhydrate

En opérant comme décrit dans l'exemple 2b/ mais en utilisant la 4-(6-chloropyrid-2-yl)-1,2,3,6-tétrahydropyridine au lieu de la 4-(3-trifluorométhylphényl)-1,2,3,6-tétrahydropyridine, on obtient le composé du titre. P.f. (chlorhydrate) 230-32°C.

### EXEMPLE 8

### Chlorhydrate de 1-[2,2-(4,4'-difluorodiphényl)éthyl]-4-(3-trifluorométhylphényl)-1,2,3,6-tétrahydropyridine

En opérant comme décrit dans l'exemple 2 mais en utilisant la 4,4'-difluorobenzophénone au lieu de la 4,4'-dichlorobenzophénone on obtient la 1-[2,2-(4,4'-difluorodiphényl)éthyl]-4-(3-trifluorométhylphényl)-1,2,3,6-tétrahydropyridine. On prépare le chlorhydrate à l'aide d'une solution d'acide chlorhydrique dans de l'isopropanol. On obtient le composé du titre. P.f. 173-175°C.

### EXEMPLE 9

### Chlorhydrate de 1-[2,2-(4,4'-dichlorodiphényl)éthyl]-4-(2-trifluorométhylphényl)-1,2,3,6-tétrahydropyridine

### 9a/ Chlorhydrate de 4-hydroxy-4-(2-trifluorométhylphényl)-pipéridine.

On mélange 3,25 g (0,135 mole) de Mg avec une pointe de spatule de I₂ et on y ajoute goutte à goutte une solution de 30,4 g (0,135 mole) de 2-bromo-1-trifluorométhylbenzène dans 125 ml de THF. On agite pendant une heure à la température ambiante et on y ajoute goutte à goutte 10,1 g (0,041 mole) de benzylpipéridone. On agite pendant 1 heure à la température ambiante et on ajoute au mélange une solution saturée en chlorure d'ammonium. On extrait à l'éther éthylique, on sèche la phase organique et on évapore le solvant sous pression réduite. On purifie le produit par chromatographie sur colonne de gel de silice en éluant avec un mélange cyclohexane/acétate d'éthyle. On obtient 6,8 g de 1-benzyl-4-hydroxy-4-(2-trifluorométhylphényl)-pipéridine qu'on hydrogène dans 75 ml d'éthanol à 95% à pH acide par ajout d'acide chlorhydrique, à l'aide de 0,7 g de Pd/C à 10 %, en chauffant à la température de 60°C pendant 8 heures. On filtre le catalyseur et on obtient ainsi 2,1 g du produit du titre. P.f. 247-251°C.

### 9b/ Chlorhydrate de 4-(2-trifluorométhylphényl)-1,2,3,6-tétrahydropyridine.

On dissout 2,0 g (0,007 mole) du produit de l'étape précédente dans 12 ml d'acide acétique glaciale. On y ajoute goutte à goutte 3 ml d'acide sulfurique concentré et on chauffe à 100 °C pendant deux heures. On verse dans de la glace, on ajoute au mélange une solution concentrée de NaOH jusqu'à pH basique et on extrait au chlorure de méthylène. On sèche la phase organique et on évapore le solvant sous pression réduite. On reprend le produit dans 15 ml d'isopropanol et on obtient la 4-(2-trifluorométhylphényl)-1,2,3,6-tétrahydropyridine. On prépare le chlorhydrate à l'aide d'une solution d'acide chlorhydrique dans de l'isopropanol. On obtient 0,9 g du composé du titre. P.f. 213-215°C.

### 9c/ Chlorhydrate de 1-[2,2-(4,4'-dichlorodiphényl)éthyl]-4-(2-trifluorométhylphényl)-1,2,3,6-tétrahydropyridine

A la température de 0/+5°C on mélange 0,5 g (0,0022 mole) du produit de l'étape précédente sous forme de base, 9 ml de méthanol, 0,33 ml d'acide acétique glacial et 0,2 g d'acétate de sodium anhydre. A la même température on ajoute au mélange 0,5 g (0,0019 mole) de α,α-(4,4'-dichlorodiphényl)acétaldéhyde préparé selon l'exemple 2a/ puis 0,33 g de cyanoborohydrure de sodium, on agite pendant 1,5 heures à basse température, puis à la température ambiante pendant une nuit. On ajoute goutte à goutte 2,1 ml d'acide chlorhydrique concentré, on laisse agiter pendant 15 minutes, on évapore le méthanol et on reprend le résidu dans un mélange acétate d'éthyle/solution aqueuse de NH₄OH diluée. On sèche la phase organique, on y ajoute une solution d'isopropanol saturée en acide chlorhydrique et on évapore le solvant. On obtient 0,48 g du composé du titre qu'on cristallise dans de l'acétate d'éthyle. P.f. 229-230°C.

## Revendications

1. Composé de formule (I) 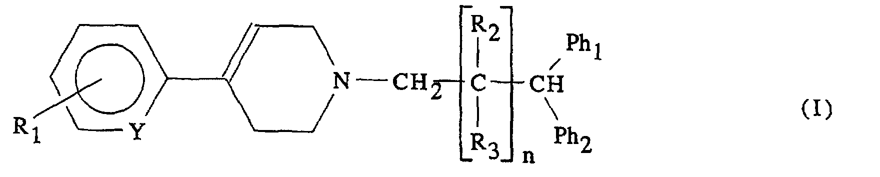 dans laquelle
Y représente -CH- ou -N-;
R₁ représente un halogène, un groupe CF₃, (C₁-C₄)alkyle ou (C₁-C₄)alcoxyle;
R₂ et R₃ représentent chacun l'hydrogène ou un (C₁-C₃)alkyle;
n représente 0 ou 1
Ph₁ et Ph₂ représentent chacun indépendamment un groupe phényle ; un groupe phényle substitué en position 2, 3 ou 4 par un atome de fluor, de chlore ou par un groupe méthyle, éthyle, *n*-propyle, *i*-propyle, *n*-butyle, *i*-butyle, *s*-butyle, *t*-butyle, trifluorométhyle, cyano, méthoxy, méthylthio, méthylsulfonyle, éthoxy, éthylthio, éthylsulfonyle, (C₁-C₃)alcoxycarbonyle ou di(C₁-C₃)alkylaminocarbonyle ; un groupe phényle disubstitué dans les positions 2, 4; 3, 4; 3, 5 ou 2, 6 par un atome de chlore, de fluor, ou par un groupe méthyle, éthyle, trifluorométhyle, cyano ou méthoxy; ou un groupe phényle trisubstitué dans les positions 3, 4, 5; 2, 4, 5 ou 2, 4, 6 par un atome de chlore, de fluor, ou par un groupe méthyle, éthyle, trifluorométhyle, cyano ou méthoxy.
ainsi que ses sels et solvates et ses sels d'ammonium quaternaire.

2. Composé selon la revendication 1, dans lequel Y est CH et R₁ est CF₃ ou Y est N et R₁ est Cl.

3. Composé selon la revendication 1 ou 2, dans lequel R₂ et R₃ sont tous les deux des hydrogènes.

4. Composé selon la revendication 1 de formule (I') 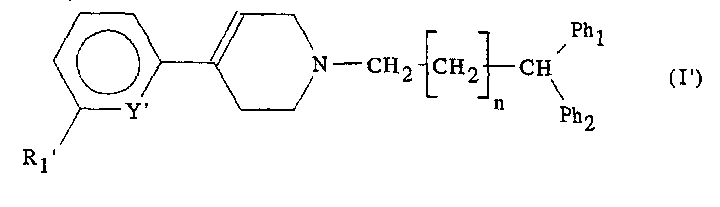 dans laquelle R₁' est CF₃ et Y' est CH ou bien R₁' est Cl et Y' est N, et n, Ph₁ et Ph₂ sont tels que définis pour les composés (I) dans la revendication 1, ainsi que ses sels et solvates et ses sels d'ammonium quaternaire.

5. Composé selon l'une quelconque des revendications 1 à 4, dans lequel Ph₁ et Ph₂ sont identiques.

6. Composé selon la revendication 1 choisi parmi la 1-(2,2-diphényléthyl)-4-(3-trifluorométhylphényl)-1,2,3,6-tétrahydropyridine; la 1-[2,2-(4,4'-dichlorodiphényl)éthyl]-4-(3-trifluorométhylphényl)-1,2,3,6-tétrahydro-pyridine; la 1-[2,2-(3,3'-bistrifluorométhyldiphényl)éthyl]-4-(3-tifluorométhylphényl)-1,2,3,6-tétrahydropyridine; la 1-[2,2-(4,4'-diméthoxydiphényl)éthyl]-4-(3-trifluorométhylphényl)-1,2,3,6-tétrahydropyridine; la 1-[2-(4-fluorophényl)-2-phényléthyl]-4-(3-trifluorométhylphényl)-1,2,3,6-tétrahydropyridine; la 1-(3,3-diphénylpropyl)-4-(3-trifluorométhylphényl)-1,2,3,6-tétrahydropyridine; la 1-[2,2-(4,4'-dichlorodiphényl)éthyl]-4-(6-chloropyrid-2-yl)-1,2,3,6-tétrahydropyridine et leurs sels, solvates ou sels d'ammonium quaternaire.

7. Procédé pour la préparation des composé de formule (I) selon la revendication 1, de leurs sels ou solvates et de leurs sels d'ammonium quaternaire, **caractérisé en ce que**:
(a) on fait réagir une aryl-1,2,3,6-tétrahydropyridine de formule (II) 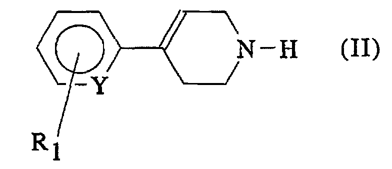 dans laquelle Y et R₁ sont tels que définis pour les composés (I) dans la revendication 1, avec un acide de formule (III) 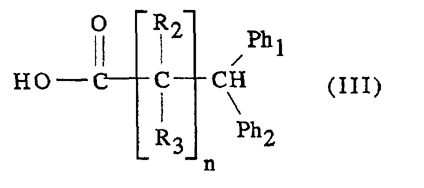 dans laquelle n, R₂, R₃, Ph₁ et Ph₂ sont tels que définis pour les composés (I) dans la revendication 1, ou l'un de ses dérivés foncionnels,
(b) on réduit le carbonyle intermédiaire de formule (IV), 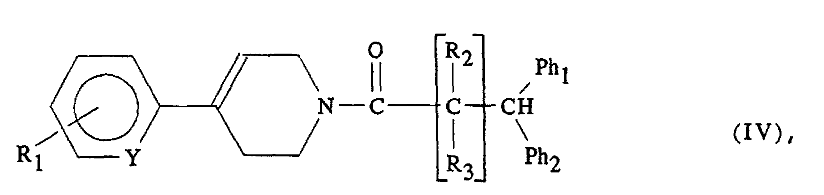 et
(c) on isole le composé de formule (I) ainsi obtenu et, éventuellement on le transforme en l'un de ses sels ou solvates ou l'un de ses sels d'ammonium quaternaire.

8. Composition pharmaceutique contenant en tant que principe actif un composé selon l'une quelconque des revendications 1 à 6.

9. Composition pharmaceutique contenant, en tant que principes actifs, un composé selon l'une des revendications 1 à 6 et un composé indiqué dans le traitement symptomatique de la démence sénile du type Alzheimer (DAT) ou leurs sels pharmaceutiquement acceptables.

## Claims

1. Compound of formula (I): 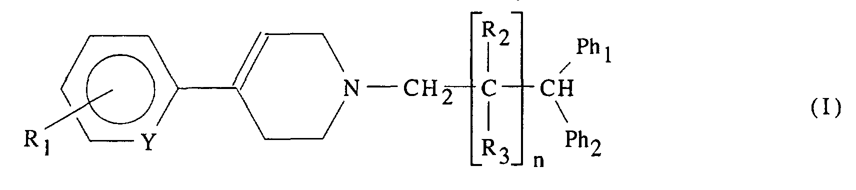 in which:
Y is -CH- or -N-;
R₁ is a halogen or a CF₃, (C₁-C₄)alkyl or (C₁-C₄)alkoxy group;
R₂ and R₃ are each hydrogen or a (C₁-C₃)alkyl;
n is 0 or 1; and
Ph₁ and Ph₂ are each independently a phenyl group; a phenyl group substituted in the 2-, 3- or 4-position by a fluorine or chlorine atom or by a methyl, ethyl, *n*-propyl, *i*-propyl, *n*-butyl, *i*-butyl, *s*-butyl, *t*-butyl, trifluoromethyl, cyano, methoxy, methylthio, methylsulfonyl, ethoxy, ethylthio, ethylsulfonyl, (C₁-C₃)alkoxycarbonyl or di(C₁-C₃)alkylaminocarbonyl group; a phenyl group disubstituted in the 2,4-, 3,4-, 3,5- or 2,6-positions by a chlorine or fluorine atom or by a methyl, ethyl, trifluoromethyl, cyano or methoxy group; or a phenyl group trisubstituted in the 3,4,5-, 2,4,5- or 2,4,6-positions by a chlorine or fluorine atom or by a methyl, ethyl, trifluoromethyl, cyano or methoxy group.
and its salts and solvates and its quaternary ammonium salts.

2. Compound according to claim 1 in which Y is CH and R₁ is CF₃, or Y is N and R₁ is Cl.

3. Compound according to claim 1 or 2 in which R₂ and R₃ are both hydrogens.

4. Compound according to claim 1 of formula (I'): 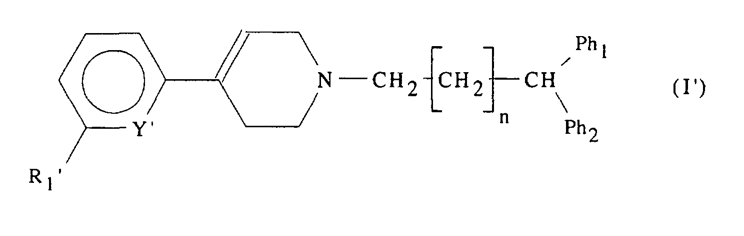 in which R₁' is CF₃ and Y' is CH, or R₁' is Cl and Y' is N, and n, Ph₁ and Ph₂ are as defined for the compounds (I) in claim 1, and its salts and solvates and its quaternary ammonium salts.

5. Compound according to anyone of claims 1 to 4 in which Ph₁ and Ph₂ are identical.

6. Compound according to claim 1 selected from 1-(2,2-diphenylethyl)-4-(3-trifluoromethylphenyl)-1,2,3,6-tetrahydropyridine; 1-[2,2-(4,4'-dichlorodiphenyl)-ethyl]-4-(3-trifluoromethylphenyl)-1,2,3,6-tetrahydropyridine; 1-[2,2-(3,3'-bistrifluoromethyldiphenyl)ethyl]-4-(3-trifluoromethylphenyl)-1,2,3,6-tetrahydropyridine; 1-[2,2-(4,4'-dimethoxydiphenyl)ethyl]-4-(3-trifluoromethylphenyl)-1,2,3,6-tetrahydropyridine; 1-[2-(4-fluorophenyl)-2-phenylethyl]-4-(3-trifluoromethylphenyl)-1,2,3,6-tetrahydropyridine; 1-(3,3-diphenylpropyl)-4-(3-trifluoromethylphenyl)-1,2,3,6-tetrahydropyridine; 1-[2,2-(4,4'-dichlorodiphenyl)ethyl]-4-(6-chloropyrid-2-yl)-1,2,3,6-tetrahydropyridine; and their salts, solvates or quaternary ammonium salts.

7. Method of preparing the compounds of formula (I) according to claim 1, their salts or solvates and their quaternary ammonium salts, **characterized in that**:
(a) an aryl-1,2,3,6-tetrahydropyridine of formula (II): 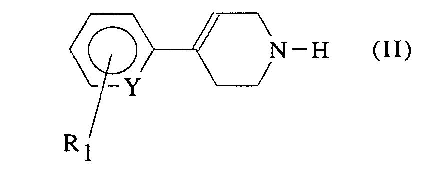 in which Y and R₁ are as defined for the compounds (I) in claim 1, is reacted with an acid of formula (III): 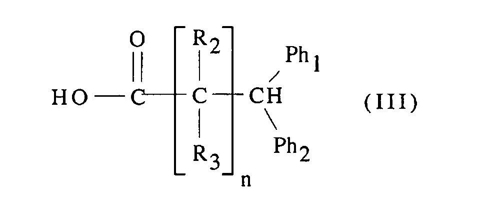 in which n, R₂, R₃, Ph₁ and Ph₂ are as defined for the compounds (I) in claim 1, or one of its functional derivatives;
(b) the intermediate carbonyl of formula (IV): 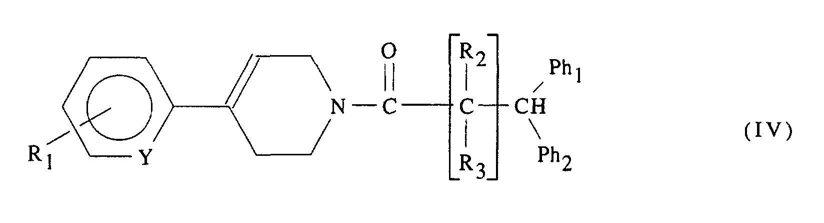 is reduced; and
(c) the resulting compound of formula (I) is isolated and optionally converted to one of its salts or solvates or one of its quaternary ammonium salts.

8. Pharmaceutical composition containing, as the active principle, a compound according to any one of claims 1 to 6.

9. Pharmaceutical composition containing, as the active principles, a compound according to one of claims 1 to 6 and a compound indicated in the symptomatic treatment of senile dementia of the Alzheimer type (DAT), or their pharmaceutically acceptable salts.

## Patentansprüche

1. Verbindung der Formel (I) 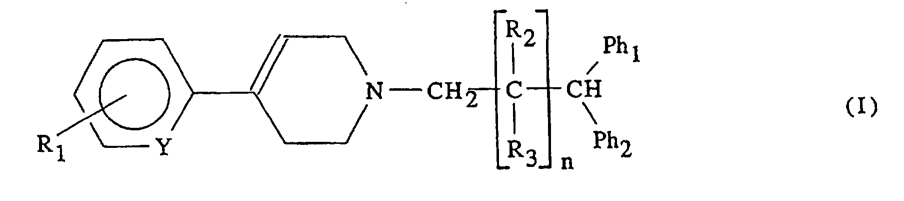 worin
Y -CH- oder -N- darstellt;
R₁ ein Halogen, CF₃, (C₁-C₄)-Alkyl oder (C₁-C₄)-Alkoxy darstellt;
R₂ und R₃ jeweils Wasserstoff oder (C₁-C₃)-Alkyl darstellen;
n für 0 oder 1 steht,
Ph₁ und Ph₂ jeweils unabhängig voneinander Phenyl; in Position 2, 3 oder 4 durch ein Fluor- oder Chloratom oder durch Methyl, Ethyl, n-Propyl, i-Propyl, n-Butyl, i-Butyl, s-Butyl, t-Butyl, Trifluormethyl, Cyano, Methoxy, Methylthio, Methylsulfonyl, Ethoxy, Ethylthio, Ethylsulfonyl, (C₁-C₃)-Alkoxycarbonyl oder Di-(C₁-C₃)-alkylaminocarbonyl substituiertes Phenyl; in den Positionen 2, 4; 3,4; 3,5 oder 2,6 durch ein Chlor- oder Fluoratom oder durch Methyl, Ethyl, Trifluormethyl, Cyano oder Methoxy disubstituiertes Phenyl; oder in den Positionen 3,4,5; 2,4,5 oder 2,4,6 durch ein Chlor- oder Fluoratom oder durch Methyl, Ethyl, Trifluormethyl, Cyano oder Methoxy trisubstituiertes Phenyl darstellen;
sowie die Salze und Solvate und die quaternären Ammoniumsalze derselben.

2. Verbindung nach Anspruch 1, worin Y für CH und R₁ für CF₃ steht oder Y für N und R₁ für Cl steht.

3. Verbindung nach Anspruch 1 oder 2, worin R₂ und R₃ beide Wasserstoff darstellen.

4. Verbindung nach Anspruch 1 der Formel (I') 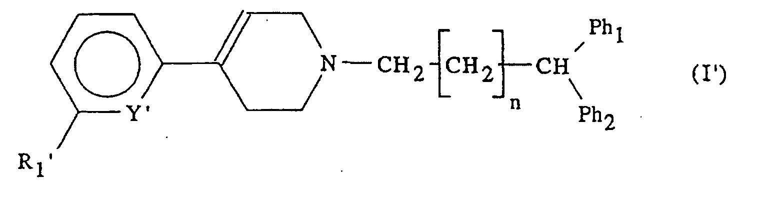 worin R₁' für CF₃ und Y' für CH steht oder aber R₁' für Cl und Y' für N steht und n, Ph₁ und Ph₂ wie in Anspruch 1 für die Verbindungen (I) definiert sind, sowie die Salze und Solvate und die quaternären Ammoniumsalze derselben.

5. Verbindung nach einem der Ansprüche 1 bis 4, worin Ph₁ und Ph₂ identisch sind.

6. Verbindung nach Anspruch 1, ausgewählt aus 1-(2,2-Diphenylethyl)-4-(3-trifluormethylphenyl)-1,2,3,6-tetrahydropyridin; 1-[2,2-(4,4'-Dichlordiphenyl)ethyl]-4-(3-trifluormethylphenyl)-1,2,3,6-tetrahydropyridin; 1-[2,2-(3,3'-Bistrifluormethyldiphenyl)ethyl]-4-(3-trifluormethylphenyl)-1,2,3,6-tetrahydropyridin; 1-[2,2-(4,4'-Dimethoxydiphenyl)ethyl]-4-(3-trifluormethylphenyl)-1,2,3,6-tetrahydropyridin; 1-[2-(4-Fluorphenyl)-2-phenylethyl]-4-(3-trifluormethylphenyl)-1,2,3,6-tetrahydropyridin; 1-(3,3-Diphenylpropyl)-4-(3-trifluormethylphenyl)-1,2,3,6-tetrahydropyridin; 1-[2,2-(4,4'-Dichlordiphenyl)ethyl]-4-(6-chlorpyrid-2-yl)-1,2,3,6-tetrahydropyridin und die Salze und Solvate und die quaternären Ammoniumsalze derselben.

7. Verfahren zur Herstellung der Verbindungen der Formel (I) nach Anspruch 1, der Salze oder der Solvate und der quaternären Ammoniumsalze derselben, **dadurch gekennzeichnet, dass**
(a) ein Aryl-1,2,3,6-Tetrahydropyridin der Formel (II) 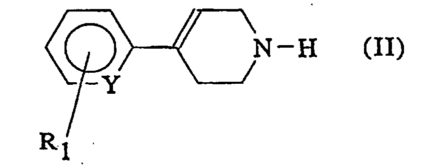 worin Y und R₁ wie in Anspruch 1 für die Verbindungen (I) definiert sind, mit einer Säure der Formel (III) 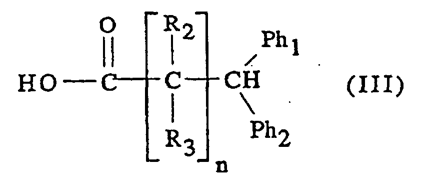 worin n, R₂, R₃, Ph₁ und Ph₂ wie in Anspruch 1 für die Verbindungen (I) definiert sind, oder einem ihrer funktionellen Derivate zur Umsetzung gebracht wird,
(b) das intermediäre Carbonyl der Formel (IV) 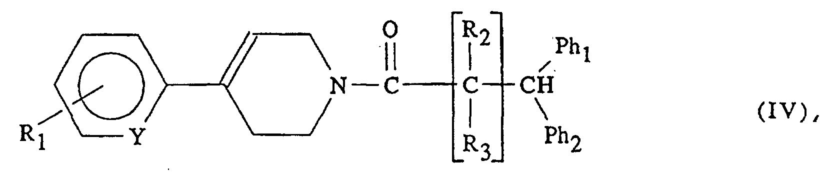 reduziert wird, und
(c) die so erhaltene Verbindung der Formel (I) isoliert und gegebenenfalls in eines ihrer Salze oder Solvate oder eines ihrer quaternären Ammoniumsalze übergeführt wird.

8. Pharmazeutische Zusammensetzung, die als Wirkstoff eine Verbindung nach einem der Ansprüche 1 bis 6 enthält.

9. Pharmazeutische Zusammensetzung, die als Wirkstoffe eine Verbindung nach einem der Ansprüche 1 bis 6 und eine Verbindung, die bei der symptomatischen Behandlung von seniler Demenz vom Alzheimer-Typ (DAT) induziert ist, oder pharmazeutisch annehmbare Salze derselben enthält.
